# EUROPÄISCHE PATENTSCHRIFT

(11) **EP 0 732 325 B1**
(45) Veröffentlichungstag und Bekanntmachung des Hinweises auf die Patenterteilung: **15.12.1999**
(21) Anmeldenummer: 96103711.6
(22) Anmeldetag: 09.03.1996
(51) Int. Cl.: C07D 213/48

(54) **Verfahren zur Herstellung von 3-Formylpyridin**
Process for the preparation of 3-formylpyridine
Procédé pour la préparation de 3-formylpyridine

(30) Priorität: 16.03.1995 DE 19509552
(43) Veröffentlichungstag der Anmeldung: 18.09.1996
(73) Patentinhaber: BASF Aktiengesellschaft, 67063 Ludwigshafen (DE)
(72) Erfinder: Schnurr, Werner, Dr., D-67273 Herxheim (DE); Fischer, Rolf, Dr., D-69121 Heidelberg (DE); Wulff-Döring, Joachim, Dr., 67227 Frankenthal (DE); Hesse, Michael, Dr., 67105 Schifferstadt (DE); Götz, Norbert, Dr., 67547 Worms (DE); Maywald, Volker, Dr., 67069 Ludwigshafen (DE)

(56) Entgegenhaltungen:
- EP-A- 0 150 961
- EP-A- 0 290 096
- EP-A- 0 343 640
- US-A- 4 328 373

## Beschreibung

Die vorliegende Erfindung betrifft ein Verfahren zur Herstellung von 3-Formylpyridin durch Umsetzung von Pyridin-3-carbonsäure mit Wasserstoff in der Gasphase in Gegenwart von Zirkon-dioxid und Lanthan enthaltenden Katalysatoren.

Aus der US-A-3,935,265 ist bekannt, daß man Alkylester aromatischer Carbonsäuren bei 400 bis 600°C an Al₂O₃ mit Wasserstoff hydrieren kann. Beispielsweise wird Benzoesäuremethylester mit einer Selektivität von 37 % (Umsatz: 39 %) zu Benzaldehyd umgesetzt. Weiterhin werden z.B. Ru/Sn- (EP-A-539 274), Manganoxid- (EP-A-290 096, US-A-4,585,899), Eisenoxid- (EP-A-304 853), Vanadiumoxid und/oder Titandioxid- (US-A-4,950,799, EP-A-414 065), Cu/Y₂O₃- (US-A-4,585,900), Cr₂O₃/ZrO₂- (EP-A-150 961, EP-A-343 640), Cr₂O₃- (US-A-5 306 845) oder Lanthanid-oxide/ Al₂O₃-Katalysatoren (US-A-4,328,373, EP-A-101 111) für die Hydrierung von aromatischen und heterocyclischen Carbonsäuren eingesetzt.

Bei den bekannten Hydrierverfahren werden in den meisten Fällen, zum Teil bedingt durch sehr hohe Hydriertemperaturen, nur unbefriedigende Ausbeuten und Selektivitäten erzielt.

Der vorliegenden Erfindung lag daher die Aufgabe zugrunde, den zuvor genannten Nachteilen abzuhelfen.

Demgemäß wurde ein neues und verbessertes Verfahren zur Herstellung von 3-Formylpyridin gefunden, welches dadurch gekennzeichnet ist, daß man Pyridin-3-carbonsäure mit Wasserstoff in der Gasphase bei Temperaturen von 200 bis 450°C und Drücken von 0,1 bis 20 bar in Gegenwart eines Katalysators, dessen katalytisch aktive Masse 80 bis 99,9 Gew.-% Zirkonoxid und 0,1 bis 20 Gew.-% Lanthan enthält, umsetzt.

Das erfindungsgemäße Verfahren läßt sich wie folgt durchführen:

Die erfindungsgemäße Hydrierung der Pyridin-3-carbonsäure mit Wasserstoff in Gegenwart eines Katalysators, dessen katalytisch aktive Masse 60 bis 99,9, insbesondere 80 bis 99,9 Gew.-% Zirkonoxid und 0,1 bis 20 Gew.-% Lanthan enthält, wird in der Regel bei Temperaturen von 200 bis 450°C, bevorzugt 250 bis 400°C, besonders bevorzugt 300 bis 380°C und Drücken von 0,1 bis 20 bar, bevorzugt 0,7 bis 5 bar, besonders bevorzugt Atmosphären-druck (Normaldruck) durchgeführt. Die erforderliche Temperatur und der erforderliche Druck sind abhängig von der Katalysator-aktivität und der thermischen Stabilität von Edukt und Produkt.

Als Katalysatoren eignen sich Trägerkatalysatoren, bevorzugt Vollkatalysatoren von Zirkondioxid in kubischer, tetragonaler oder monokliner Phase, bevorzugt in monokliner Phase, die mit Lanthan dotiert sind. Die katalytisch aktive Masse enthält in der Regel 80 bis 99,9 Gew.-%, bevorzugt 90 bis 99,9 Gew.-%, besonders bevorzugt 92 bis 99 Gew.-% Zirkonoxid und 0,1 bis 20 Gew.-% Lanthan, bevorzugt 0,1 bis 10 Gew.-% Lanthan, besonders bevorzugt 1 bis 8 Gew.-% Lanthan-(III)-oxid. Die Dotierung erfolgt in der Regel durch Tränken des Zirkonoxids mit Salzlösungen (wäßrig oder alkoholisch) des Lanthans.

Der Katalysator kann zusätzlich weitere Dotierungen (z.B. Chrom, Eisen, Yttrium, Hafnium, Mangan) in Mengen von 0,001 bis 10 Gew.-% enthalten. Bevorzugt sind Katalysatoren ohne solche Zusätze.

Die BET-Oberfläche des Zirkonoxids kann in weiten Grenzen schwanken, beträgt 20 bis 150 m²/g, besonders bevorzugt 40 bis 120 m²/g.

Derartige Katalysatoren werden in bekannter Weise z. B. durch Tränken vorgeformter Träger wie Pellets, Kugeln oder Stränge, Trocknen und Calcinieren hergestellt.

Die bevorzugt verwendeten Trägerkatalysatoren zeigen über einen längeren Zeitraum hohe Aktivität. Desaktivierte Katalysatoren lassen sich durch Behandlung mit molekularen Sauerstoff enthaltenden Gasen, z.B. Luft, bei Temperaturen von 350 bis 500°C regenerieren.

Im allgemeinen hält man eine Katalysatorbelastung von 0,01 bis 10, vorzugsweise 0,01 bis 3 kg Carbonsäure je kg Katalysator und Stunde ein.

Die Wasserstoffkonzentration im Eingangsgas richtet sich nach der Carbonsäure(ester)konzentration. Das Molverhältnis von Wasserstoff zu Carbonsäure beträgt in der Regel 2:1 bis 100:1, bevorzugt 10:1 bis 70:1. Als Wasserstoffquelle kann auch Ameisensäure eingesetzt werden.

Vorteilhaft kann auch der Zusatz eines inerten Verdünnungsmittels sein. In der Regel werden Stickstoff, Wasser oder gasförmige, unter den Reaktionsbedingungen inerte Verbindungen wie z.B. Kohlenwasserstoffe, Aromaten oder Ether verwendet.

Die Umsetzung kann in der Gasphase, kontinuierlich als Festbettreaktion mit fest angeordnetem Katalysator, beispielsweise in Sumpf- oder Rieselfahrweise oder als Wirbelbettreaktion mit in auf- und abwirbelnder Bewegung befindlichem Katalysator durchgeführt werden. Bevorzugt ist das Arbeiten im Festbett.

Zur Steigerung der Selektivität können bei der Hydrierung gebildete Nebenprodukte, z.B. Alkohole, in die Synthese zurückgeführt werden.

Mit Hilfe des erfindungsgemäßen Verfahrens ist es möglich, 3-Formylpyridin auf einfache Weise selektiv herzustellen.

3-Formylpyridin eignet sich als Geruchs- und Geschmacksstoff bzw. als Zwischenprodukt z.B. für Pharma- und Pflanzenschutzwirkstoffe (Ullmann's Encyclopedia of Industrial Chemistry, Vol. A3, Seite 469 bis 474).

### Beispiele

### Katalysatorherstellung

### Beispiel 1

Monoklines Zirkondioxid in Form von Tabletten (BET-Oberfläche: 53 m²/g, Katalysator A) oder Strängen (BET-Oberfläche: 92 m²/g, Katalysator B) wurde mit einer wäßrigen Lanthannitrat-Lösung unter guter Durchmischung getränkt und 2 h bei Raumtemperatur gehalten. Anschließend wurde der Katalysator 15 Stunden bei 120°C getrocknet und anschließend 2 bis 4 Stunden bei 400 bis 500°C mit Luft getempert.

Der so hergestellten Katalysator hatte einen Lanthan-Gehalt von 3 Gew.-%.

### Beispiel 2

Pro Stunde wurden 50 g 10 %ige wäßrige Pyridin-3-carbonsäure-Lösung mit 1501/h Wasserstoff verdampft und bei 320°C über 128 g Katalysator A in Rieselfahrweise geleitet. Der gasförmige Reaktionsaustrag wurde in Kühlfallen kondensiert und gaschromatographisch analysiert. Die 3-Formylpyridin-Ausbeute betrug 72 % (Umsatz 100 %).

## Patentansprüche

1. Verfahren zur Herstellung von 3-Formylpyridin, dadurch gekennzeichnet, daß man Pyridin-3-carbonsäure mit Wasserstoff in der Gasphase bei Temperaturen von 200 bis 450°C und Drükken von 0,1 bis 20 bar in Gegenwart eines Katalysators, dessen katalytisch aktive Masse 80 bis 99,9 Gew.-% Zirkonoxid mit einer BET-Oberfläche von 20 bis 150 m²/g und 0,1 bis 20 Gew.-% Lanthan enthält, umsetzt.

2. Verfahren nach Anspruch 1, dadurch gekennzeichnet, daß der Katalysator als katalytisch aktive Masse 90 bis 99,9 Gew.-% Zirkonoxid und 0,1 bis 10 Gew.-% Lanthan enthält.

3. Verfahren nach einem der Ansprüche 1 oder 2, dadurch gekennzeichnet, daß der Katalysator als katalytisch aktive Masse 92 bis 99 Gew.-% Zirkonoxid und 1 bis 8 Gew.-% Lanthan-(III)-oxid enthält.

4. Verfahren nach Anspruch 1, dadurch gekennzeichnet, daß das Zirkondioxid monoklin ist.

5. Verfahren nach Anspruch 1, dadurch gekennzeichnet, daß das Zirkondioxid eine BET-Oberfläche von 40 bis 120 m²/g hat.

6. Verfahren zur Herstellung von 3-Formylpyridin nach Anspruch 1, dadurch gekennzeichnet, daß das Molverhältnis von Wasserstoff zu Pyridin-3-carbonsäure 10:1 bis 70:1 beträgt.

7. Verfahren nach Anspruch 1, dadurch gekennzeichnet, daß man die Umsetzung im Festbett durchführt.

## Claims

1. A process for the preparation of 3-formylpyridine which comprises reacting pyridine-3-carboxylic acid with hydrogen in the gas phase at from 200 to 450°C and from 0.1 to 20 bar in the presence of a catalyst whose catalytically active material contains from 80 to 99.9% by weight of zirconium oxide having a BET surface area of from 20 to 150 m²/g and from 0.1 to 20% by weight of lanthanum.

2. A process as claimed in claim 1, wherein the catalyst contains, as catalytically active material, from 90 to 99.9% by weight of zirconium oxide and from 0.1 to 10% by weight of lanthanum.

3. A process as claimed in either of claims 1 and 2, wherein the catalyst contains, as catalytically active material, from 92 to 99% by weight of zirconium oxide and from 1 to 8% by weight of lanthanum(III) oxide.

4. A process as claimed in claim 1, wherein the zirconium dioxide is monoclinic.

5. A process as claimed in claim 1, wherein the zirconium dioxide has a BET surface area of from 40 to 120 m²/g.

6. A process for the preparation of 3-formylpyridine as claimed in claim 1, wherein the molar ratio of hydrogen to pyridine-3-carboxylic acid is from 10:1 to 70:1.

7. A process as claimed in claim 1, wherein the reaction is carried out in a fixed bed.

## Revendications

1. Procédé pour préparer la 3-formylpyridine, caractérisé par le fait que l'on fait réagir l'acide pyridine-3-carboxylique avec l'hydrogène en phase gazeuse à des températures de 200 à 450°C sous des pressions de 0,1 à 20 bar, en présence d'un catalyseur dont la masse catalytique active contient 80 à 99,9% en poids d'un oxyde de zirconium à une surface BET de 20 à 150 m²/g et 0,1 à 20% en poids de lanthane.

2. Procédé selon la revendication 1, caractérisé par le fait que le catalyseur contient en tant que masse catalytique active 90 à 99,9% en poids d'oxyde de zirconium et 0,1 à 10% en poids de lanthane.

3. Procédé selon l'une des revendications 1 ou 2, caractérisé par le fait que le catalyseur contient en tant que masse catalytique active 92 à 99% en poids d'oxyde de zirconium et 1 à 8% en poids d'oxyde de lanthane-III.

4. Procédé selon la revendication 1, caractérisé par le fait que le dioxyde de zirconium est monoclinique.

5. Procédé selon la revendication 1, caractérisé par le fait que le dioxyde de zirconium a une surface BET de 40 à 120 m²/g.

6. Procédé de préparation de la 3-formylpyridine selon la revendication 1, caractérisé par le fait que le rapport molaire hydrogène/acide pyridine-3-carboxylique va de 10 : 1 à 70 : 1.

7. Procédé selon la revendication 1, caractérisé par le fait que la réaction est réalisée en lit fixe.
